**Europäisches Patentamt**

(19) **European Patent Office**

**Office européen des brevets**

(11) Veröffentlichungsnummer : **0 173 723 B1**

(12) **EUROPÄISCHE PATENTSCHRIFT**

(45) Veröffentlichungstag der Patentschrift :
11.10.89

(51) Int. Cl.⁴ : **A 61 F  2/24**

(21) Anmeldenummer : **85901357.5**

(22) Anmeldetag : **08.03.85**

(86) Internationale Anmeldenummer :
**PCT/DE 85/00074**

(87) Internationale Veröffentlichungsnummer :
**WO/8504094 (26.09.85 Gazette 85/21)**

(54) PROTHESE ZUM ERSATZ VON AORTENKLAPPEN.

(30) Priorität : **09.03.84 DE 3409005**

(43) Veröffentlichungstag der Anmeldung :
**12.03.86 Patentblatt 86/11**

(45) Bekanntmachung des Hinweises auf die Patenterteilung : **11.10.89 Patentblatt 89/41**

(84) Benannte Vertragsstaaten :
**CH DE FR GB LI SE**

(56) Entgegenhaltungen :
**EP--A-- 0 079 844
FR--A-- 1 355 214
FR--A-- 2 345 140
US--A-- 4 406 022**

(73) Patentinhaber : SIEVERS, Hans-Hinrich, Dr.
**Wilhelmshavenerstr. 1
D-2300 Kiel 1 (DE)**

(72) Erfinder : SIEVERS, Hans-Hinrich, Dr.
**Wilhelmshavenerstr. 1
D-2300 Kiel 1 (DE)**

(74) Vertreter : Tönnies, Jan G., Dipl.-Ing.
**Niemannsweg 133
D-2300 Kiel 1 (DE)**

**Beschreibung**

Die Erfindung betrifft eine Prothese zum Ersatz der Aortenklappe nach dem Oberbegriff des Anspruchs 1, wie sie aus der FR-A-1 355 214 vorbekannt ist.

Bei der vorbekannten Prothese sind die Flügelklappen als elastische « Segel » ausgebildet, was in bezug auf Dauerhaftigkeit und Verläßlichkeit Probleme mit sich bringt.

Aus der US-A-4 406 022 ist weiter eine dreiflüglige Prothese bekannt, bei der die Flügelklappen starr sind. Diese Flügelklappe ist jedoch strömungstechnisch ungünstig, da die Flügel gestreckt ausgebildet sind.

Aus der EP-A-0 079 844 ist weiter eine Prothese zum Ersatz der Aortenklappen bekannt. Diese Prothese ist mit lediglich zwei Flügelklappen versehen, deren obere und untere Teile zueinander stumpfwinklig verlaufen, wobei der obere Teil sich von der Mittelachse aus gesehen von dem unteren Teil weg weisend erstreckt.

Der Erfindung liegt die Aufgabe zugrunde, eine Flügelklappe mit starren Flügeln zu schaffen, die schnell und zuverlässig schließt.

Erfindungsgemäß wird diese Aufgabe durch die im kennzeichnenden Teil des Anspruchs 1 angegebenen Merkmale gelöst. Der Unteranspruch gibt vorteilhafte Ausgestaltungen der Erfindung an.

Die Erfindung wird im folgenden anhand einer Zeichnung erläutert. Dabei zeigt :

Fig. 1 perspektivisch die Prothese in Offenstellung,
Fig. 2 perspektivisch die Prothese in Sperrstellung,
Fig. 3 in Draufsicht die Prothese in Offenstellung,
Fig. 4 in Draufsicht die Prothese in Sperrstellung,
Fig. 5 eine Abwandlung der Prothese gemäß Fig. 1-4 bei Draufsicht in Sperrstellung,
Fig. 6 einen Schwenkflügel im Längsschnitt, sowie.

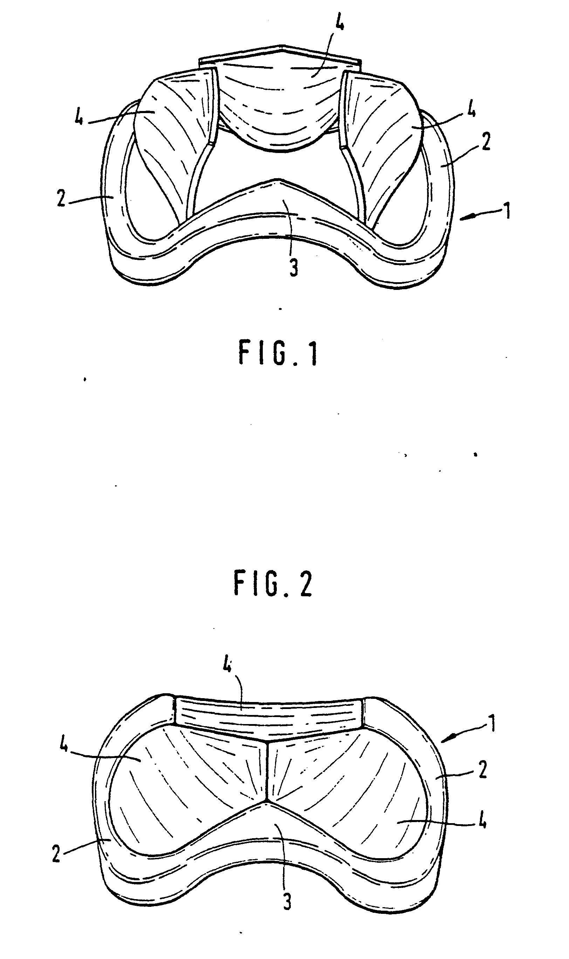

Die in den Figuren 1 bis 4 in ihrer Gesamtheit dargestellte Prothese besitzt einen starren Ringkörper 1, der aus drei Bogenabschnitten 2 zusammengesetzt ist. Jeder Bogenabschnitt 2 ist in sich im wesentlichen eben und zum Lot auf dem Mittelpunkt der durch die Scheitelpunkte der Bogenabschnitte definierten, gedachten Grundfläche schräg gestellt, wobei die Bogenenden 3 nach innen weisen und die Ebenen der Bogenabschnitte 2 mit dem Lot einen Winkel von etwa 40-50°, beispielsweise 45°, einschließen. Die Krümmungsform der Bogenabschnitte kann ein Halbkreisbogen (wie in Figur 1 bis 4 dargestellt), ein Halbellipsenbogen, ein Parabelbogen oder ein sonstwie gekrümmter Bogen sein, der dem Verlauf der Anheftungszonen der natürlichen Aortenklappensegel an der Aortenwand weitgehend entspricht.

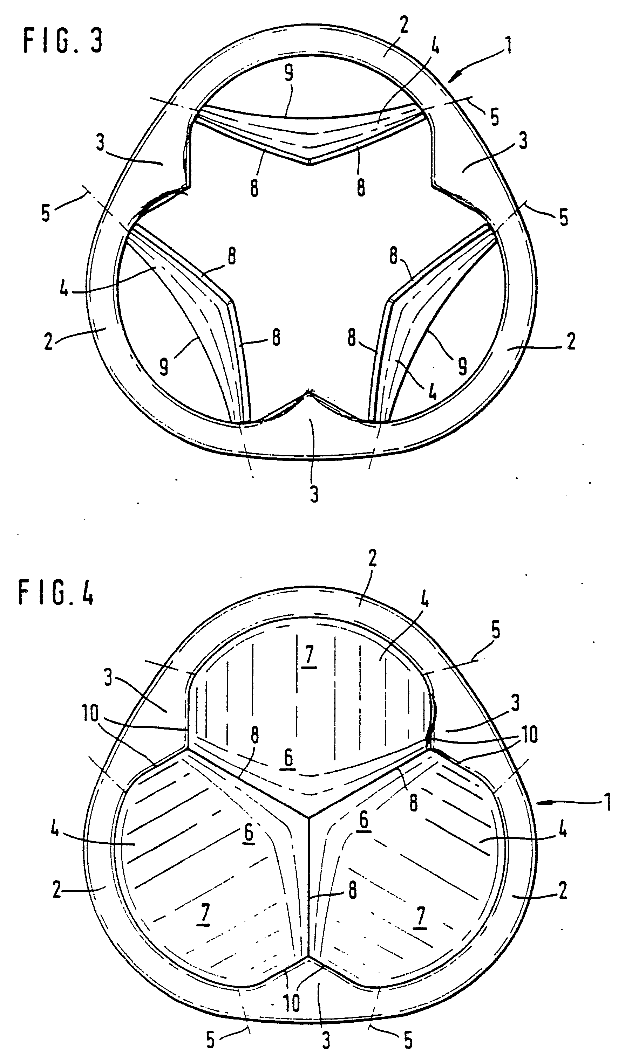

In jedem Bogenabschnitt 2 ist ein Schwenkflügel 4 gelagert, dessen Schwenkachse 5 nahe den Bogenenden 3 so durch den Schwenkflügel 4 verläuft, daß das obere Flügelteil 6 eine größere Fläche besitzt als das untere Flügelteil 7. Dadurch wird gewährleistet, daß die Schwenkflügel 4 aufgrund der gegebenen Druckverhältnisse während der Systole der linken Herzkammer öffnet und eine stabile Lage gemäß Figur 1 bzw. Figur 3 einnehmen und daß sie beim Beginn der Diastole schnell und sicher in die Sperrstellung zurückschwenken.

Normalerweise sind die drei Bogenabschnitte 2 und die drei Schwenkflügel 4 jeweils übereinstimmend ausgebildet, so daß die Prothese in bezug auf das Lot im Mittelpunkt der Grundfläche des Ringkörpers 1 eine dreizählige Drehsymmetrie besitzt. Dies ist der Darstellung der Figuren 1 bis 4 zugrundegelegt. Es ist aber ebenso auch eine unsymmetrische Prothese möglich, bei der die Schräglage der Bogenabschnitte, deren Krümmungsform und/oder die Form und Größe der Schwenkflügel unterschiedlich ausgebildet ist. Dies kann in einzelnen Fällen zweckmäßig sein, beispielsweise um anatomische Besonderheiten zu berücksichtigen oder das Durchflußverhalten unsymmetrisch zu gestalten.

Unabhängig davon, ob die Prothese symmetrisch ist oder nicht, muß das untere Flügelteil 7 jedes Schwenkflügels 4 so geformt sein, daß es in der Sperrstellung der Schwenkflügel 4 den zugeordneten Bogenabschnitt 2 dichtend abdeckt, und das obere Flügelteil 6 jedes Schwenkflügels muß zwei stumpfwinklig zueinander verlaufende Innenkanten 8 aufweisen, entlang denen sich die Schwenkflügel in der Sperrstellung dichtend gegeneinanderlegen können. In dem in Figur 1 bis 4 dargestellten Ausführungsbeispiel sind die Innenkanten der Schwenkflügel 4 gerade ebene Kanten, die in der Sperrstellung stumpf aufeinander stoßen, bei einer bevorzugten Ausführungsform sind die Innenkanten 8 ballig ausgebildet, was ein zuverlässiges Schließen auch bei geringfügigem Lagerspiel gewährleistet.

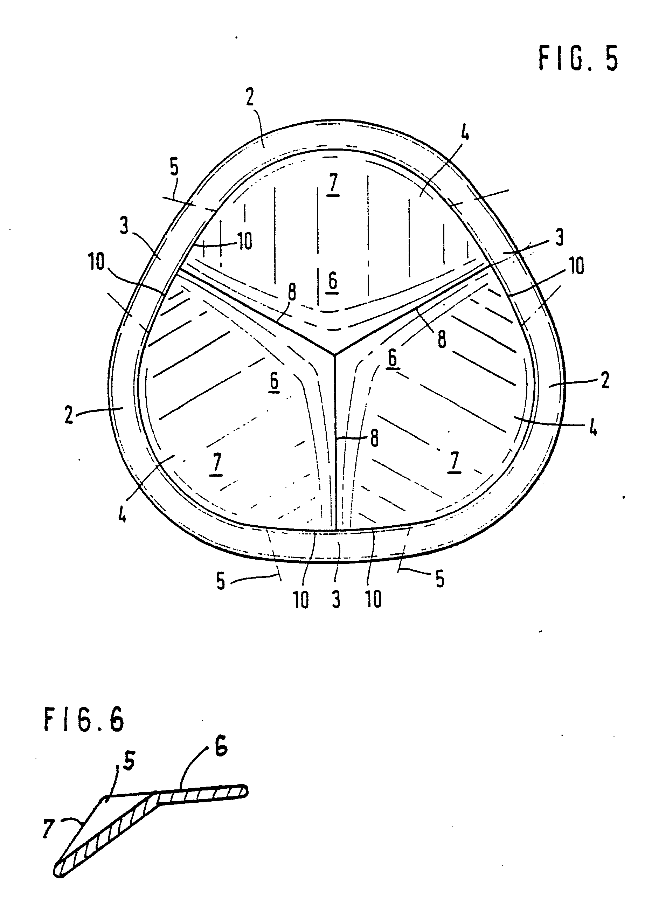

Die Schwenkachsen 5 sind im Bereich der äußersten Enden der Bogenenden 3 angeordnet, so daß der Anfangsteil des Randes der oberen Flügelteile 6 eine Dichtlinie 10 mit dem Ringkörper 1 besitzt. Diese Dichtlinie 10 kann unterschiedlich verlaufen, wie die Ausbildungen gemäß Figur 4 im Vergleich mit Figur 5 zeigen. Auf die Funktion der Prothese wirkt sich der unterschiedliche Verlauf der Dichtlinie 10 praktisch nicht aus.

Die Schwenkflügel 4 sind im einfachsten Fall dünne Scheiben (Figur 6), bei denen der obere Flügelteil 6 im wesentlichen eben ausgebildet ist und der untere Flügelteil 7 mit einer quer zur Schwenkachse verlaufenden, eine Einbuchtung in Richtung auf das Lot bildenden Vertiefung versehen ist.

Fig. 6 verdeutlicht, daß die Flügelklappen nicht gestreckt ausgebildet sind, sondern derart abgewinkelt, daß der obere, in der Sperrstellung an den benachbarten oberen Teilen 6 der benachbarten Flügelklappen 4 anliegende obere Flügelteil winklig zu dem unteren, in der Sperrstellung den Bogenabschnitt abdeckenden unteren Teil verläuft, so daß der obere Flügelteil (6) in einer zu der Grundebene und zu der jeweiligen Schwenkachse rechtwinkligen Schnittebene von der Mittelachse der Prothese aus gesehen stumpfwinklig zu dem unteren Flügelteil verläuft.

## Patentansprüche

1. Prothese zum Ersatz der Aortenklappen, mit einem als Ventilsitz ausgebildeten starren Ringkörper, der mittels eines an seiner Außenseite angeordneten Textilsaumes mit der Aortenwand chirurgisch vernähbar ist und der die Form von drei an ihren Enden (3) miteinander verbundenen Bogenabschnitten (2) besitzt, deren Ebenen jeweils in spitzem Winkel zu der durch die Scheitelpunkte der Bogenabschnitte definierten Grundfläche aufeinander zu verlaufen, und mit drei jeweils einem der Bogenabschnitte (2) zugeordneten, am Ringkörper gelagerten Flügelklappen (4), die jeweils zwei in einem stumpfen Winkel aufeinander zu verlaufende Innenkanten aufweisen deren Ausbildung so getroffen ist, daß sich in der Sperrstellung jede Innenkante jedes Schwenkflügels (4, 20) der zugekehrten Innenkante des benachbarten Schwenkflügels (4, 20) annähert, und die je nach Schwenkstellung den Durchlaß durch die Innenöffnung des Ringkörpers freigeben oder sperren, dadurch gekennzeichnet,

daß die Flügelklappen (4) als Schwenkflügel (4) ausgebildet, und mit einer im Bereich der Bogenabschnitte (2) gelagerten, durch den Flügel verlaufenden Schwenkachse (5) versehen sind,

daß die Flügelklappen (4) mit einem in der Sperrstellung den Bogenabschnitt (2) abdeckenden kleineren Teil (7) und einem in der Sperrstellung an den benachbarten Flügelklappen (4) anliegenden größeren Teil (6) ausgebildet sind, und

daß der obere Flügelteil (6) in einer zu der durch die Scheitelpunkte der Bogenabschnitte (2) definierten Grundebene und zu der jeweiligen Schwenkachse (5) rechtwinkligen Schnittebene von der Mittelachse der Prothese aus gesehen stumpfwinklig zu dem unterem Flügelteil (7) verläuft.

2. Prothese nach Anspruch 1, dadurch gekennzeichnet, daß das größere Teil (6) im wesentlichen eben ausgebildet und das kleinere Teil (7) mit einer schwachen Krümmung ausgebildet ist.

## Claims

1. Prothesis for replacement of the aorta valves, with a rigid annular body being formed as an valve seat, which can be sutured surgically by means of a fabric seam to the wall of the aorta and which has the form of three arcs (2) connected with one another at their tips (3), the plane of said arcs point towards one another in an acute angle with the plane defined by the vertex points of the arcs, and with three leaflets (4) each assigned to one of the arcs and supported by the annular body, said leaflets comprise each two inner edges pointing in an obtuse angle to each other, whose inner edges are formed such that in the closed position each inner edge of each leaflet (4, 20) approaches the edge of the neighbouring leaflet (4, 20), and the position of the leaflets determines whether the inner opening of the annular body is open or closed, characterised in

that the leaflets (4) are formed as pivot wings (4) with their pivot axis supported in the region of their respective arc and running through the wing,

that the leaflets (4) are formed with a smaller part (7) which covers the arc (2) when in the closed position and with a bigger part (6) which abuts the neighbouring leaflet (4) when in the closed position, and

that the upper part of the leaflet (6) runs in a sectional plane which is defined by the vertex points of the arcs (2) and which is at a right angle with the pivot axis (5) in an obtuse angle seen from the middle axis of the prothesis.

2. Prothesis according to claim 1, characterised in that the bigger part (6) is formed substantially flat and the smaller part is slightly curved.

## Revendications

1. Prothèse pour remplacer les valvules de l'aorte avec un bâti rigide annulaire formé comme siège de soupape qui peut être suturée à la paroi de l'aorte à l'aide d'une bordure en textile attachée au côté extérieur et qui a la forme de trois sections arquées (2) liées l'une à l'autre à leurs bouts (3) dont les plans respectifs sont orientés vers le plan de base défini par les sommets des sections arquées en angle de pointe, et avec trois clapets en forme d'aile allant respectivement avec une des sections arquées (2) et supportés au bâti angulaire (4) qui montrent respectivement deux bords intérieurs orientés l'un à l'autre en angle obtus qui sont construits à ce que, en position de blocage chaque bord intérieur de chaque aile basculant s'approche du bord intérieur opposé de l'aile basculant voisin et qui, selon la position du basculement, ouvrent ou bloquent le passage à travers l'ouverture intérieure du bâti annulaire caractérisés par les points suivants :

les clapets en forme d'aile (4) sont construits comme des ailes basculantes (4) et sont munis d'un axe de basculement supporté aux sections arquées (2) et allant à travers l'aile,

les clapets en forme d'aile (4) sont construits avec une partie plus petite (7) couvrant la section arquée (2) en position de blocage et une partie plus grande (6) ajustée aux clapets voisins (4) en position de blocage, et

la partie supérieure de l'aile (6) est orientée dans un plan d'intersection formant un angle droit avec le plan de base défini par les sommets des sections arquées (2) et avec l'axe de basculement respectif (5) vers la partie inférieure de l'aile (7) en angle obtus vu de l'axe central de la prothèse.

2. Prothèse d'après la revendication 1 caractérisée par le fait que la partie plus grande (6) est essentiellement construite d'une façon plane et la partie plus petite (7) avec un courbement léger.

FIG.1

FIG.2

FIG. 3

FIG. 4

FIG. 5

FI6.6